# EUROPÄISCHE PATENTSCHRIFT

(11) **EP 3 041 526 B1**
(45) Veröffentlichungstag und Bekanntmachung des Hinweises auf die Patenterteilung: **04.03.2020**
(21) Anmeldenummer: 14758958.4
(22) Anmeldetag: 04.09.2014
(51) Int. Cl.: A61M 3/02, A61B 17/3203

(54) **HANDSTÜCK ZUR REINIGUNG VON WUNDEN**
HANDPIECE FOR THE CLEANING OF WOUNDS
PIÈCE À MAIN DESTINÉE À NETTOYER DES BLESSURES

(30) Priorität: 06.09.2013 EP 13183379
(43) Veröffentlichungstag der Anmeldung: 13.07.2016
(73) Patentinhaber: MEDAXIS AG, 6340 Baar (CH)
(72) Erfinder: SCHUG, Martin, CH-6045 Meggen (CH); STEINER, Claudio, CH-6340 Baar (CH); WIDMER, Beat, CH-6005 Luzern (CH)
(74) Vertreter: Clerc, Natalia
(86) Internationale Anmeldenummer: PCT/EP2014/068853
(87) Internationale Veröffentlichungsnummer: WO 2015/032866

(56) Entgegenhaltungen:
- WO-A1-82/03316
- WO-A1-2013/084945
- US-A- 5 037 431
- US-A- 5 380 300
- US-A- 6 099 494
- US-A1- 2006 264 851
- US-A1- 2007 100 300

## Beschreibung

### TECHNISCHES GEBIET

Die vorliegende Erfindung betrifft ein Handstück, einen porösen Körper sowie ein Verfahren zur Reinigung von Wunden mit einem Fluidstrahl.

### STAND DER TECHNIK

Als Wundreinigung / Debridement und Wundspülung / Wundtoilette bezeichnet man medizinische Vorgehen zur Entfernung von infiziertem, geschädigtem oder abgestorbenem (nekrotischem) Gewebe aus Geschwüren, Verbrennungs- und anderen Wunden oder bei Organzerfall. Es existieren zahlreiche generelle Ansätze für die Wundreinigung, z.B. mechanisch-scharfe unter der Verwendung eines Skalpells oder eines scharfen Löffels, enzymatische oder chemische, autolytische, biochirurgische, mechanische unter der Verwendung von Pads und mit Fluidstrahl.

Die EP 2 251 142 zeigt ein Handstück zur Reinigung von Wunden mit einem Hochdruck-Mikro-Fluidstrahl, wobei eine Austrittsöffnung am vorderen Ende des Handstücks angeordnet ist, durch welche ein Fluidstrahl austreten kann.

Je nach Konsistenz, Lage, Alter und Beschaffenheit der Beläge von Wunden muss der Fluidstrahl unterschiedlich lange und intensiv appliziert werden, um bei maximaler Schonung des Gewebes dennoch ein genügend wirksames und genügend schonendes Abtrennen und Entfernen der Wundbeläge zu gewährleisten.

Oftmals reicht der Fluidstrahl jedoch alleine nicht aus, um den gewünschten Reinigungseffekt zu erzielen. In diesem Fall werden zusätzliche mechanische Reinigungselemente, wie z.B. Pads, Skalpelle oder scharfe Löffel eingesetzt. Dies ist jedoch unhandlich, da der Operateur dazu eine zweite Hand benötigt. Die Hand kann seine eigene oder die eines Assistenten sein. Dies verkompliziert die Wundreinigung. Zudem ist diese Behandlung meistens für den Patienten schmerzhaft und besteht die Gefahr, dass das Gewebe unnötig verletzt wird.

Bei der Wundreinigung mittels eines Fluidstrahls werden Beläge oder Partikel aus der Wunde entfernt, wobei Aerosole entstehen. Es ist wichtig sicherzustellen, dass die Umgebung so wenig wie möglich durch diese Aerosole kontaminiert wird, da dies eine Gefährdung für den Patienten oder das Operationspersonal darstellt. Aus dem Stand der Technik sind zahlreiche Verfahren bekannt, die die Kontaminierung der Umgebung durch die Aerosole reduzieren oder verhindern. Solche Verfahren, wie z.B. Flüssigkeitsrückführung, abschirmendes Behandlungszelt mit Abzugsschleuse oder die Anordnung des Fluidstrahls und der Absaugung in einer Abdeckhaube. Die genannten Verfahren sind aufwändig, da nicht nur eine Flüssigkeitszufuhr sondern auch eine - Rückführung vorhanden sein muss.

Beispiele für Wundreinigungsvorrichtungen mit Flüssigkeitsrückführung sind in WO 2008/074284 und WO 2004/037095 offenbart. US 6,099,494 offenbart eine Wundreinigungsvorrichtung mittels Fluidstrahl, bei der eine schützende Hülle einer Düse vorsteht. US 2006/264851 offenbart eine Reinigungsvorrichtung, bei der ein poröser Körper die Austrittsöffnungen einer Reinigungsflüssigkeit umgibt.

### DARSTELLUNG DER ERFINDUNG

Es ist daher eine Aufgabe der vorliegenden Erfindung, die Reinigung einer Wunde zu verbessern und eine Verbreitung der Aerosole zu verhindern.

Diese Aufgabe wird erfindungsgemäss durch ein Handstück mit den Merkmalen des Patentanspruchs 1 gelöst.

Das erfindungsgemässe Handstück zum Reinigen von Wunden durch einen Fluidstrahl umfasst einen Grundkörper mit einem vorderen Ende mit einer Austrittsöffnung zum Austritt des Fluidstrahls. Am vorderen Ende des Handstücks ist ein poröser Körper vorhanden, welcher die Austrittsöffnung umgibt und ihr in Richtung des Fluidstrahlauslasses vorsteht und welcher einen Raum bildet, der vom Fluidstrahl ungehindert passierbar ist. Im vorderen Ende des Handstücks ist eine Düse angeordnet, welche die Austrittsöffnung bildet. Weiterhin ist das Handstück steif ausgebildet.

Das erfindungsgemässe Handstück kombiniert die Vorteile der Reinigung und Behandlung mit einem Fluidstrahl, insbesondere einem Mikro-Fluidstrahl, mit den Vorteilen einer mechanischen Wundreinigung in einem einfachen und kostengünstigen Handstück.

Im erfindungsgemässen Verfahren zum Reinigen von Wunden mittels eines derartigen Handstücks wird die Wunde gleichzeitig mit einem aus dem Handstück austretenden Fluidstrahl und einem am Handstück angeordneten porösen Körper bearbeitet.

Je nach Anwendung kann der Druck des Fluidstrahls unterschiedlich eingestellt sein. So sind gewisse Wunden mittels hohen Drucks behandelbar, während andere mit geringerem Druck behandelbar sind.

Durch die gleichzeitige Beaufschlagung der Wunde mit einem Fluid, insbesondere einer wässrigen Lösung bzw. einer Behandlungslösung, wie z.B. einer sterile Kochsalzlösung, und der mechanischen Bearbeitung der Wunde mit dem porösen Körper wird das Gewebe, insbesondere das vitale und granulierende Gewebe, nach wie vor, im Vergleich zu allen mechanisch scharfen Methoden optimal geschont. Die kombinierte Anwendung von Fluidstrahl und dem mechanisch wirkenden porösen Körper erhöht nicht nur die Reinigungswirkung sondern aktiviert und stimuliert das Gewebe zusätzlich. Dies fördert die Wundheilung und einen beschleunigten Wundverschluss, wodurch die Gesamtbehandlungskosten reduziert werden.

Die Wundreinigung ist verbessert und die Behandlungszeit verkürzt, bei zusätzlicher Förderung der Wundheilung. Gleichzeitig ist ein Schutz vor Aerosolen vorhanden, so dass insbesondere bei der Reinigung von kleineren Wunden auf eine Abdeckung bzw. ein Schutzzelt verzichtet werden kann.

Die mechanische Bearbeitung kann lokal gezielt und nur solange angewandt werden, wie unbedingt notwendig. Es ist nicht notwendig, dass während der gesamten Beaufschlagung mit dem Fluid auch mechanisch bearbeitet wird.

Da der Körper porös ist, können entstehende Aerosole im Körper gefangen werden. Der poröse Körper kann zudem Fluide und Gewebeteilchen aufnehmen. Eine zusätzliche Absaugung kann entfallen. Da der Körper den Fluidstrahl umgibt und der Austrittsöffnung vorsteht, bildet er einen optimalen Schutz auf kleinstem Raum. Der poröse Körper kann an seinem äusseren Umfang offenporig ausgebildet sein. Sein äusserer Mantel kann jedoch auch durch eine dichte Aussenhaut gebildet sein. Beispielsweise kann er mit einer Silikonschicht oder mit einer Folie überdeckt sein. Die Aussenhaut kann auch durch eine Sprühhaut, eine Lackierung, durch Schmelzen oder andere bekannte Techniken erzeugt werden.

Es ist möglich, zusätzlich eine Absaugung vorzusehen. Diese zusätzliche Absaugung kann vermehrt Aerosole, Fluide, Biofilme oder andere in der Wunde vorhandene Stoffe ableiten. Falls eine Absaugung vorhanden ist, erfolgt diese in bevorzugten Ausführungsformen im porösen Körper. Vorzugsweise ist der poröse Körper hierfür mit der dichten Aussenhaut versehen. Die Absaugung kann durch die ohnehin vorhandenen Poren des porösen Körpers erfolgen. In bevorzugten Ausführungsformen weist der poröse Körper Saugkanäle mit grösserem Durchmesser als die Poren auf, wobei diese Saugkanäle vorzugsweise annähernd parallel zur Strahlrichtung des Fluidstrahls verlaufen. Vorzugsweise verlaufen die Saugkanäle parallel zur Mantelfläche des porösen Körpers. In weiteren Ausführungsformen sind alternativ oder zusätzlich radial verlaufende Saugkanäle vorhanden, welche in den vom porösen Körper umschlossenen Raum, auch erste Durchgangsöffnung genannt, münden. Dank der Absaugung ist der poröse Körper weniger schnell gesättigt und das Handstück lässt sich länger verwenden.

Vorzugsweise ist der Fluidstrahl ein Mikro-Fluidstrahl, insbesondere ein Hochdruck-oder Niederdruck-Mikro-Fluidstrahl. Üblicherweise beträgt der Druckbereich 1 bis 300 bar. Der Fluidstrahl ist vorzugsweise ein Mikro-Fluidstrahl, d.h. ein Fluidstrahl mit einem Durchmesser von circa 0,05 mm bis 0,15 mm bei Austritt aus der Austrittsöffnung. Üblicherweise ist der Fluidstrahl ein einfacher Vollstrahl. Es ist jedoch auch ein Kegel-, Hohlkegel- oder Flachstrahl als Einfach- oder Mehrfachstrahl einsetzbar.

Die Austrittsöffnung des Fluidstrahls ist in bevorzugten Ausführungsformen so ausgebildet, dass der Strahl annähernd parallel zur Längsmittelachse des porösen Körpers verläuft. In anderen bevorzugten Ausführungsformen verläuft er in einem Winkel zu dieser Längsmittelachse. Vorzugsweise beträgt der Winkel circa 45°. Diese im Winkel stehende Austrittsrichtung ergibt eine im Vergleich zur mittelachsparallelen Austrittsrichtung andere Bearbeitungswirkung und Abrasionswirkung der Wundoberfläche. So weist ein Fluidstrahl, welcher in einem 45° Winkel zur Längsmittelachse austritt und somit auch in diesem Winkel auf die Wundoberfläche auftrifft, eine Schälwirkung auf.

Der poröse Körper kann einstückig ausgebildet sein. Er kann auch aus mehreren Teilkörpern bestehen, welche am vorderen Ende des Handstücks angeordnet sind und zusammen einen geschlossenen, die Austrittsöffnung umgebenden Körper bilden. Der poröse Körper kann auch Unterbrüche aufweisen. Vorzugsweise ist er jedoch mehrheitlich oder vollständig geschlossen ausgebildet, so dass die Austrittsöffnung lückenlos vom porösen Körper umgeben ist.

Der poröse Körper besteht vorzugsweise aus einem schwamm-, vlies- oder gewirkeartigen Material. Vorzugsweise ist das Material synthetisch. Diese Materialien weisen für die Wundreinigung günstige mechanische Eigenschaften auf. Sie sind genügend fest, dass sie eine genügende Eigenstabilität aufweisen, aber genügend nachgiebig, dass sie beim Kontakt mit der Wunde keine Verletzungen hervorrufen. Sie zeichnen sich weiter durch eine aufgrund der Porosität vergrösserte Oberfläche aus, welche die Reinigungswirkung begünstigt.

Der poröse Körper kann mit einer desinfizierend wirkenden Beschichtung versehen sein.

Vorzugsweise ist der poröse Körper mit einer Innenfläche am vorderen Ende des Handstücks anordnungsbar. Zusätzlich oder alternativ ist er mit einer dem vorderen Ende des Handstücks zugewandten hinteren Stirnseite am Handstück anordnungsbar. Wird nur die Innenfläche zur Anordnung verwendet, so kann beispielsweise der poröse Körper über das vordere Ende des Handstücks geschoben werden. Die Nutzung der hinteren Stirnseite des porösen Körpers als Kontaktfläche vergrössert die Gesamtfläche, was z.B. bei einer stoffschlüssigen Verbindung vorteilhaft ist.

Vorzugsweise ist das vordere Ende des Handstücks transparent ausgebildet, was eine verbesserte Sicht auf die zu reinigende Wunde erlaubt.

Der poröse Körper ist vorzugsweise fix oder lösbar am vorderen Ende des Handstücks angeordnet. Eine lösbare Verbindung zwischen dem vorderen Ende des Handstücks und dem porösen Körper bietet den Vorteil, dass der poröse Körper austauschbar ist und nach Gebrauch entsorgt werden kann oder dass unterschiedliche poröse Körper für unterschiedliche Anwendungsfälle eingesetzt werden können. Beispielsweise lassen sich verschiedene poröse Körper mit demselben Handstück verwenden, welche sich in der Porosität, der Form, dem Material und/oder dem Abrasivitätsgrad voneinander unterscheiden.

Der poröse Körper kann jedoch auch form- oder kraftschlüssig mit dem vorderen Ende des Handstücks verbunden sein. Dies vereinfacht die Herstellung, da auf aufwändige Verbindungen verzichtet werden kann.

Vorzugsweise ist eine Aussenkontur des vorderen Endes grösser ausgebildet als die die Innenfläche beinhaltende Innenkontur des porösen Körpers. Dadurch ist der poröse Körper klemmend auf dem vorderen Ende des Handstücks anordnungsbar. Vorzugsweise ist der poröse Körper dennoch verschiebbar, was die Einstellung des Abstandes von der Austrittsöffnung bis zu einer Kontaktfläche des porösen Körpers ermöglicht. Somit lässt er sich beispielsweise zur mechanischen Behandlung der Wunde zur Wunde hinunterschieben und bei alleiniger Verwendung des Fluidstrahls aus dem Kontaktbereich mit der Wunde entfernen. Ein spezifisch auf den Patienten bzw. dessen Wunde abgestimmter Abstand ist somit einstellbar.

Vorzugsweise weist das vordere Ende des Handstücks eine Aussparung zur Aufnahme des porösen Körpers auf. Dies erleichtert das Anbringen des porösen Körpers am vorderen Ende des Handstücks. Im Falle einer fixen Verbindung erleichtert eine Aussparung die Positionierung und vergrössert die Kontaktfläche zwischen dem vorderen Ende des Handstücks und dem porösen Körper. Im Falle einer lösbaren Verbindung ermöglicht eine Aussparung die Ausgestaltung einer formschlüssigen Verbindung.

Vorzugsweise ist der poröse Körper mittels eines Adapters am vorderen Ende des Handstücks anordnungsbar. Der Adapter bietet den Vorteil, dass dieser zusammen mit dem porösen Körper einfach abnehmbar ist. So kann eine form- oder kraftschlüssige Verbindung zwischen dem vorderen Ende des Handstücks und dem Adapter realisiert werden. Vorzugsweise ist der Adapter so ausgestaltet, dass er das vordere Ende des Handstücks bis auf eine Austrittsöffnung vollständig abdeckt. Dies bietet den Vorteil, dass der vordere Teil nicht mit den entstehenden Aerosolen kontaminiert wird. Der Adapter kann jedoch auch so ausgestaltet sein, dass ein Bereich des vorderen Endes des Handstücks von vorne zugänglich ist.

Der Körper weist vorzugsweise eine im Wesentlichen hohlzylindrische, keglige, oder polyedrische Form auf. Zylindrische und keglige Formen sind in ihrer Herstellung und Montage einfacher und daher kostengünstiger. Polyedrische Formen können hingegen spezifisch ausgebildet werden, um z.B. unterschiedlich steife Bereiche im porösen Körper auszubilden.

Vorzugsweise weist der poröse Körper einen Kontaktbereich zum Kontakt mit einer Wunde auf, welcher sich im Wesentlichen senkrecht zur Richtung des Fluidstrahlauslasses erstreckt.

Der poröse Körper weist vorzugsweise einen Kontaktbereich zum Kontakt mit einer Wunde auf, welcher im Wesentlichen unter einem Winkel ungleich 90° zur Richtung des Fluidstrahlauslasses erstreckt. Wenn dann bei der Reinigung der Kontaktbereich an die zu reinigende Wunde angelegt wird, so trifft der Fluidstrahl unter einem Winkel auf die zu reinigende Oberfläche. Der Winkel zwischen dem Fluidstrahl und der Senkrechten auf die zu reinigende Oberfläche kann 0° bis 90° betragen. Beispielsweise können poröse Körper mit einem Winkel von 5°, 10°, 15°, 30°, 45°, 60° oder 75° dem Anwender zur Verfügung stehen.

Vorzugsweise weist der poröse Körper im Kontaktbereich eine nach aussen gerichtete, umlaufende vordere Auskragung auf. Die Auskragung erhöht die Steifigkeit des porösen Körpers im Kontaktbereich. Ebenfalls vergrössert sie den durch den porösen Körper abdeckbaren Bereich der zu reinigenden Wunde.

Der poröse Körper weist in einer weiteren bevorzugten Ausführungsform eine in einem hinteren Bereich nach innen gerichtete, umlaufende hintere Auskragung zum Eingreifen in eine entsprechende Ausnehmung des vorderen Endes auf. Dadurch wird eine einfach herzustellende lösbare Verbindung zwischen den beiden Elementen realisiert. Die Form der Auskragung muss so ausgebildet sein, dass eine im Wesentlichen formschlüssige Verbindung realisierbar ist. Die Auskragung kann z.B. eine kreisförmige, polyedrische oder schraubenförmige Form aufweisen. Sie kann jedoch auch aus mehreren aneinandergereihten Auskragungen bestehen.

Vorzugsweise ist ein wie vorgängig beschriebener poröser Körper zur Verwendung mit einem vorgängig beschriebenen Handstück zu verwenden. Das Handstück und der poröse Körper bilden eine aufeinander abgestimmte Einheit.

Der poröse Körper weist einen Adapter zur Verbindung mit dem Handstück auf. Vorzugsweise ist der Adapter mit dem porösen Körper stoffschlüssig verbunden und bildet mit ihm eine auswechselbare Einheit. Unterschiedliche Einheiten können so schnell und einfach ausgewechselt werden.

Vorzugsweise weist der poröse Körper eine erste Durchgangsöffnung auf, welche den Raum bildet. Dadurch wird der Fluidstrahl nicht vom porösen Körper abgelenkt.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Darstellung einer ersten Ausführungsform eines erfindungsgemässen Handstücks mit Fluidstrahl;
- Fig. 2: eine zentrale Schnittansicht des vorderen Endes des Handstücks mit porösem Körper gemäss Figur 1 mit Fluidstrahl;
- Fig. 3: eine perspektivische Darstellung einer zweiten Ausführungsform eines erfindungsgemässen Handstücks mit Fluidstrahl;
- Fig. 4: eine zentrale Schnittansicht des vorderen Endes des Handstücks mit porösem Körper gemäss Figur 3 mit Fluidstrahl;
- Fig. 5: eine perspektivische Darstellung einer dritten Ausführungsform eines erfindungsgemässen Handstücks mit Fluidstrahl;
- Fig. 6: eine zentrale Schnittansicht des vorderen Endes des Handstücks mit porösem Körper gemäss Figur 5 mit Fluidstrahl;
- Fig. 7: eine perspektivische Darstellung einer vierten Ausführungsform eines erfindungsgemässen Handstücks mit Fluidstrahl;
- Fig. 8: eine zentrale Schnittansicht des vorderen Endes des Handstücks mit porösem Körper gemäss Figur 7 mit Fluidstrahl;
- Fig. 9: eine zentrale Schnittansicht des vorderen Endes eines Handstücks mit porösem Körper gemäss einer weiteren Ausführungsform;
- Fig. 10: eine zentrale Schnittansicht des vorderen Endes eines Handstücks mit porösem Körper gemäss einer weiteren Ausführungsform;
- Fig. 11: eine zentrale Schnittansicht einer weiteren erfindungsgemässen Ausführungsform mit Absaugung;
- Fig. 12: eine vergrösserte Darstellung eines Ausschnitts X gemäss Figur 11;
- Fig. 13: eine zentrale Schnittansicht einer weiteren erfindungsgemässen Ausführungsform mit Absaugung;
- Fig. 14: eine zentrale Schnittansicht einer weiteren erfindungsgemässen Ausführungsform mit Absaugung;
- Fig. 15: eine perspektivische Ansicht der Ausführungsform gemäss Figur 14 von unten;
- Fig. 16: eine zentrale Schnittansicht einer weiteren erfindungsgemässen Ausführungsform mit Absaugung;
- Fig. 17: einen Schnitt durch die Ausführungsform gemäss Figur 16 entlang A-A;
- Fig. 18: eine zentrale Schnittansicht der Ausführungsform gemäss Figur 14;
- Fig. 19: einen Schnitt durch die Ausführungsform gemäss Figur 18 entlang A-A,
- Fig. 20: eine zentrale Schnittansicht einer weiteren erfindungsgemässen Ausführungsform und
- Fig. 21: eine perspektivische Darstellung der Ausführungsform gemäss Figur 20 von unten.

### BESCHREIBUNG BEVORZUGTER AUSFÜHRUNGSFORMEN

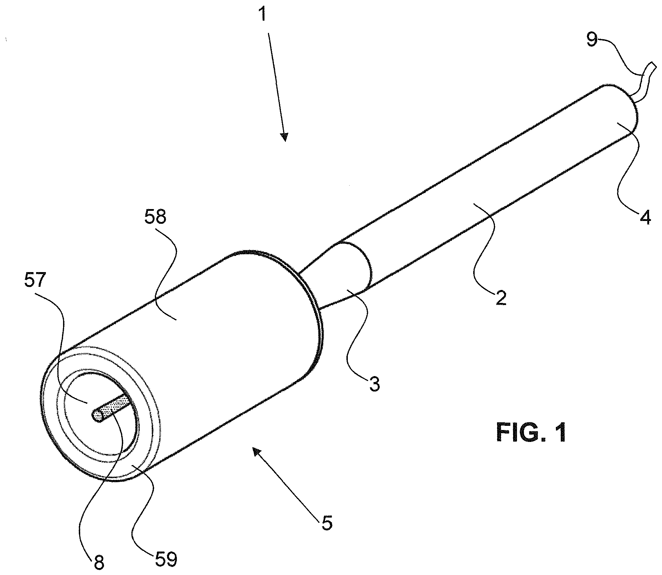

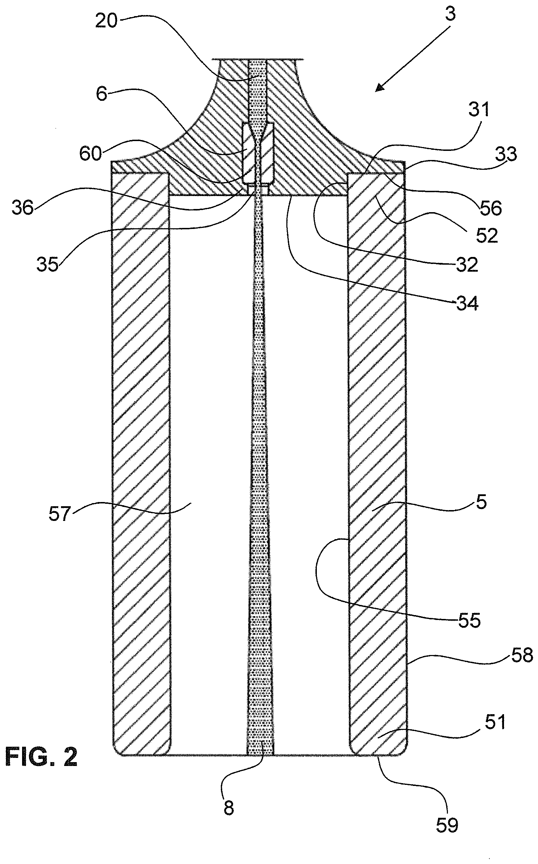

Die Figuren 1 und 2 zeigen eine perspektivische Darstellung einer ersten Ausführungsform eines erfindungsgemässen Handstücks 1 mit Fluidstrahl 8, bzw. eine zentrale Schnittansicht dessen vorderen Endes 3. Das Handstück 1 weist einen im Wesentlichen zylindrischen Grundkörper 2 zum einhändigen Halten mit einem vorderen Ende 3 und einem hinteren Ende 4 auf. Vorzugsweise ist das vordere Ende 3 transparent ausgebildet. Das vordere Ende 3 weist eine Austrittsöffnung 35 zum Austritt des Fluidstrahls 8 auf. Die Austrittsöffnung 35 ist zentral in einer ersten vorderen Stirnfläche 34 des Handstücks 1 angeordnet. Das Handstück 1 ist derart ausgebildet, dass der Fluidstrahl 8 im Wesentlichen kollinear zur Mittelachse des Grundkörpers 2 aus der Austrittsöffnung 35 austritt.

Am vorderen Ende 3 ist ein poröser Körper 5 zur sanften mechanischen Wundreinigung und als Schutzhülle angeordnet. Der poröse Körper 5 weist im Wesentlichen die Form eines Hohlzylinders mit einer ersten Durchgangsöffnung 57 auf, welche einen Raum bildet. Er umgibt mit einem hinteren Bereich 52 die Austrittsöffnung 35 und steht ihr in Richtung des Fluidauslasses vor. Die erste vordere Stirnfläche 34 weist eine Aussparung mit einer ersten axialen und einer ersten radialen Begrenzungsfläche 31, 32 zur Aufnahme des porösen Körpers 5 auf. Der poröse Körper 5 liegt mit einer Innenfläche 55 an der ersten radialen Begrenzung 32 des vorderen Endes 3 an und liegt mit einer hinteren Stirnfläche 56 an der ersten axialen Begrenzung 31 des vorderen Endes 3 an. Eine äussere Oberfläche 58 des porösen Körpers 5 ist fluchtend mit einer ersten radialen Aussenfläche 33 des vorderen Endes 3 ausgebildet.

Vorzugsweise besteht in diesem Ausführungsbeispiel eine stoffschlüssige Verbindung zwischen dem vorderen Ende 3 und dem porösen Körper 5. Eine kraft- und/oder formschlüssige Verbindung ist jedoch auch realisierbar.

Der poröse Körper 5 weist auf einer dem hinteren Bereich 52 gegenüberliegenden Seite einen Kontraktbereich 51 auf. Dieser weist eine zur ersten vorderen Stirnfläche 34 des vorderen Endes 3 parallele Kontaktfläche 59 auf, welche an die Innenfläche 55 und die äussere Oberfläche 58 angrenzt. Die Übergänge zwischen der Kontaktfläche 59 und der Innenfläche 55 bzw. der äusseren Oberfläche 58 sind vorzugsweise abgerundet.

Das vordere Ende 3 umfasst einen zentral angeordneten Fluidkanal 20 und eine damit fluchtende Düse 6, welche wiederum mit der Austrittsöffnung 35 fluchtet. Die im Wesentlichen zylindrische Düse 6 ist in bekannter Art und Weise im vorderen Ende 3 des Handstücks 1 aufgenommen und seine Position in Strahlrichtung ist durch einen vorderen Anschlag 36 bestimmt. Die Düse 6 weist einen darin zentral angeordneten Düsenkanal 60 auf. Die Ausgestaltung dieses Kanals bestimmt die Austrittsgeometrie des Fluidstrahls 8.

Die Figur 1 zeigt weiter, dass das hintere Ende 4 eine Fluidleitung 9 aufweist, wobei die Fluidleitung 9 die Versorgung des Handstücks 1 mit Fluid sicherstellt. Die nachfolgenden Ausführungsformen des erfindungsgemässen Handstücks 1 weisen ebenfalls eine Fluidleitung 9 am hinteren Ende 4 auf. Sie ist jedoch nicht dargestellt.

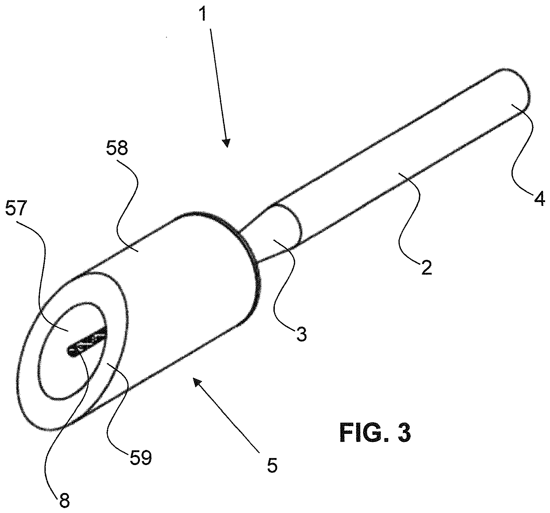

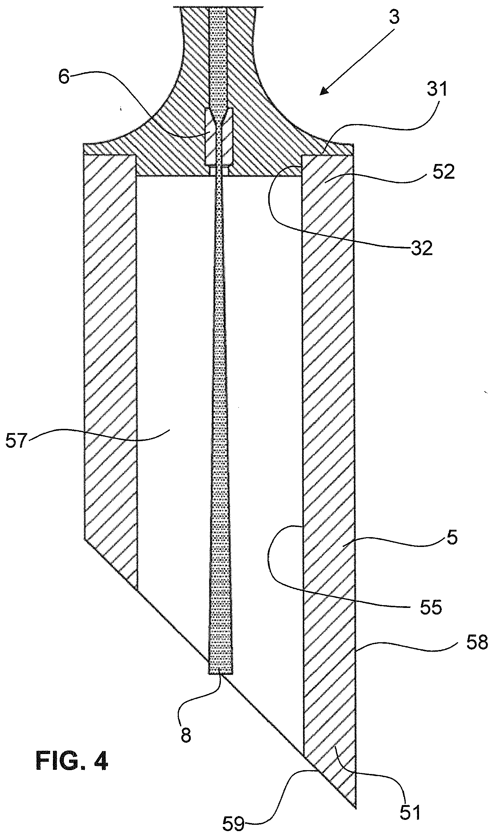

Die Figuren 3 und 4 zeigen eine perspektivische Darstellung einer zweiten Ausführungsform des erfindungsgemässen Handstücks 1 mit Fluidstrahl 8, bzw. eine zentrale Schnittansicht dessen vorderen Endes 3. Im Wesentlichen sind die erste und die zweite Ausführungsform gleich. Im Unterschied zur ersten Ausführungsform, weist der poröse Körper 5 der zweiten Ausführungsform einen abgeschrägten Kontaktbereich 51 auf und hat vorzugsweise scharfkantige Übergänge zwischen der Kontaktfläche 59 und der Innenfläche 55 bzw. der äusseren Oberfläche 58. Die Abschrägung zeigt sich darin, dass die Kontaktfläche 59 unter einem Winkel von ungleich 90° zur Strahlrichtung erstreckt. Der dargestellte Winkel entspricht in etwa 45°.

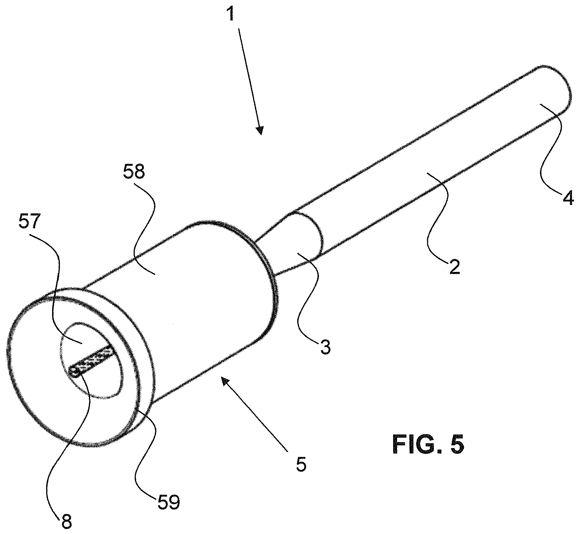

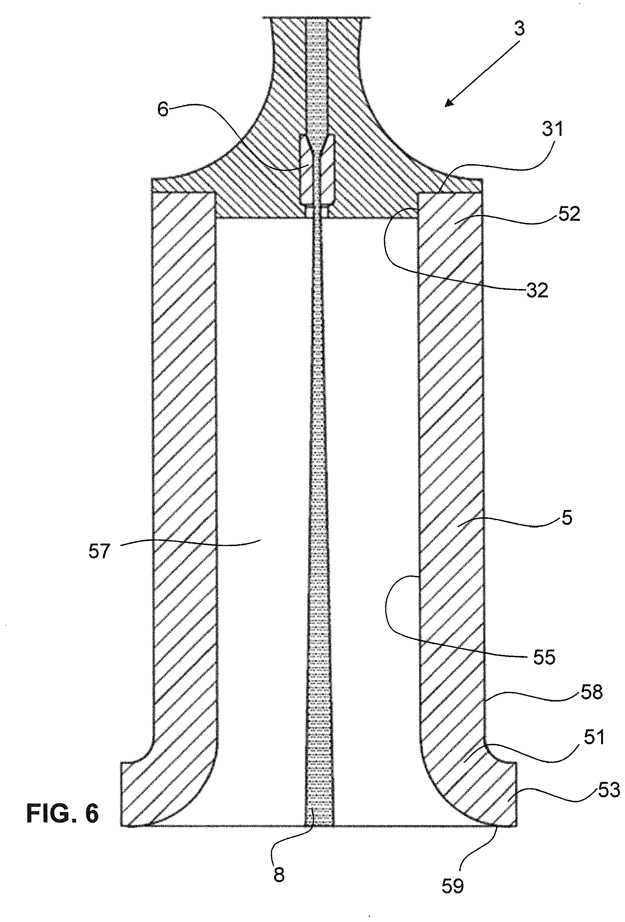

Die Figuren 5 und 6 zeigen eine perspektivische Darstellung einer dritten Ausführungsform eines erfindungsgemässen Handstücks 1 mit Fluidstrahl 8, bzw. eine zentrale Schnittansicht dessen vorderen Endes 3. Im Wesentlichen sind die erste, zweite und dritte Ausführungsform gleich. Im Unterschied zu den ersten beiden Ausführungsformen, weist der poröse Körper 5 der dritten Ausführungsform im Kontaktbereich 51 eine nach aussen gerichtete vordere Auskragung 53 auf. Der Übergang zwischen der vorderen Auskragung 53 und der Innenfläche 55 bzw. der äusseren Oberfläche 58 ist abgerundet. Die Wandstärke des porösen Körpers 5 ist gleichbleibend über seine zylindrische Länge und ändert sich im Bereich der vorderen Auskragung 53 nicht. Durch die Abrundung des Übergangs von der Kontaktfläche 59 zur Innenfläche 55 entsteht ein trichterförmiger Bereich in der ersten Durchgangsöffnung 57 im Kontaktbereich 51.

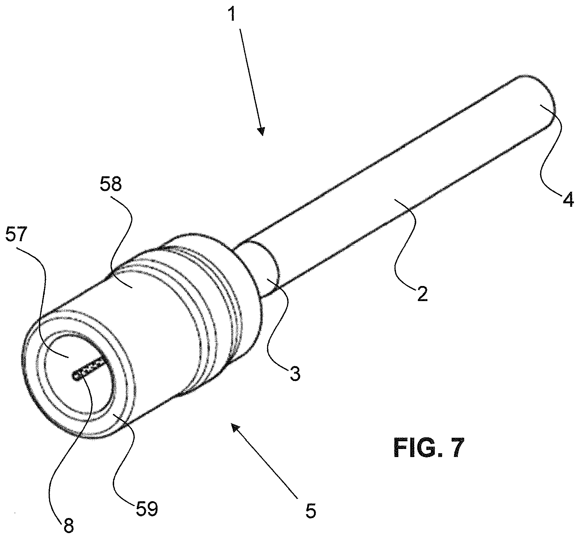

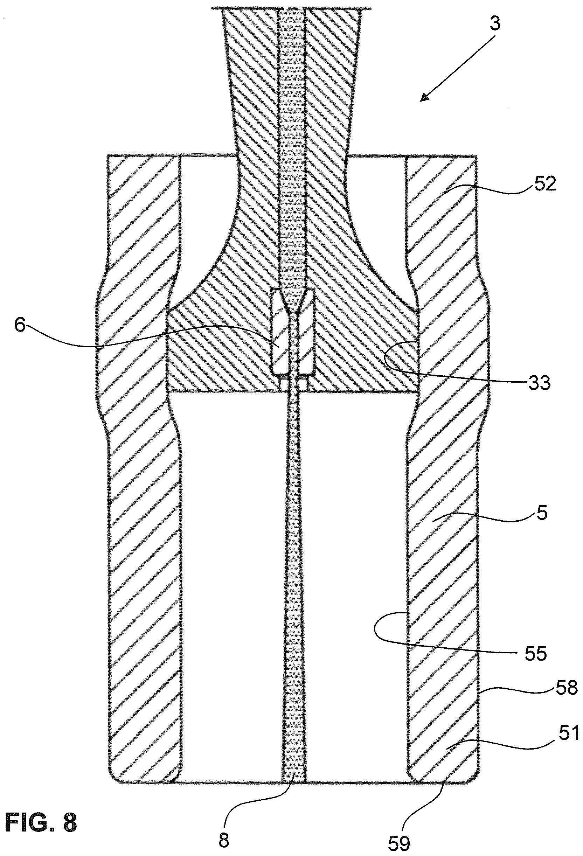

Die Figuren 7 und 8 zeigen eine perspektivische Darstellung einer vierten Ausführungsform eines erfindungsgemässen Handstücks 1 mit Fluidstrahl 8, bzw. eine zentrale Schnittansicht dessen vorderen Endes 3. Der poröse Körper 5 ist im Wesentlichen gleich ausgebildet wie derjenige der ersten Ausführungsform. Das vordere Ende 3 der vierten Ausführungsform ist jedoch unterschiedlich zu demjenigen der ersten Ausführungsform. Vorzugsweise ist das vordere Ende 3 jedoch ebenfalls transparent ausgebildet. Die erste radiale Aussenfläche 33 des vorderen Endes 3 ist so bemessen, dass diese in die erste Durchgangsöffnung 57 einführbar ist. Vorzugsweise ist der Durchmesser der ersten radialen Aussenfläche 33 etwas grösser als der Innendurchmesser der ersten Durchgangsöffnung 57. Die Innenfläche 55 ist verschiebbar, klemmend auf der ersten radialen Aussenfläche 33 des vorderen Endes 3 angeordnet. Der Abstand zwischen der Austrittsöffnung 35 und der Kontaktfläche 59 ist durch eine Relativbewegung zwischen dem vorderen Ende 3 und dem porösen Körper 5 einstellbar.

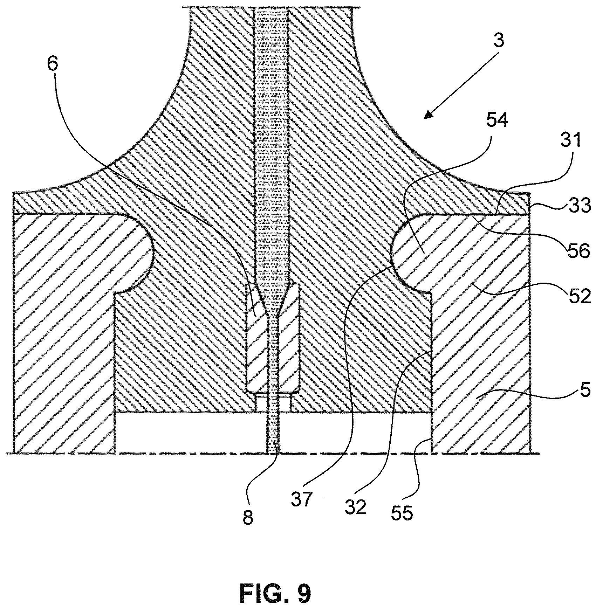

Die Figur 9 zeigt eine zentrale Schnittansicht des vorderen Endes eines Handstücks mit porösem Körper gemäss einer weiteren Ausführungsform. Im Wesentlichen zeigt die Figur 9 eine alternative lösbare Verbindung zwischen dem vorderen Ende 3 und dem porösen Körper 5 wie sie beim Handstück 1 einer ersten, einer zweiten oder einer dritten Ausführungsform anwendbar wäre. Vorteilhaft ist das vordere Ende 3 dieser Ausführungsform ebenfalls transparent ausgebildet. Der poröse Körper 5 weist im hinteren Bereich 52 eine nach innen gerichtete, umlaufende, hintere Auskragung 54 auf, welche in eine entsprechende Ausnehmung im vorderen Ende 3 eingreift und mit ihr eine lösbare Verbindung 37 bildet. Dargestellt ist eine im Querschnitt halbkreisförmige Auskragung 54. Die Innenfläche 55 des porösen Körpers 5 steht im lösbaren Kontakt mit der ersten radialen Begrenzung 32 des vorderen Endes 3 und die hintere Stirnseite 56 des porösen Körpers 5 steht im lösbaren Kontakt mit der ersten axialen Begrenzung 31 des vorderen Endes 3. Durch Ziehen am porösen Körper 5 in Strahlrichtung ist dieses vom vorderen Ende entfernbar.

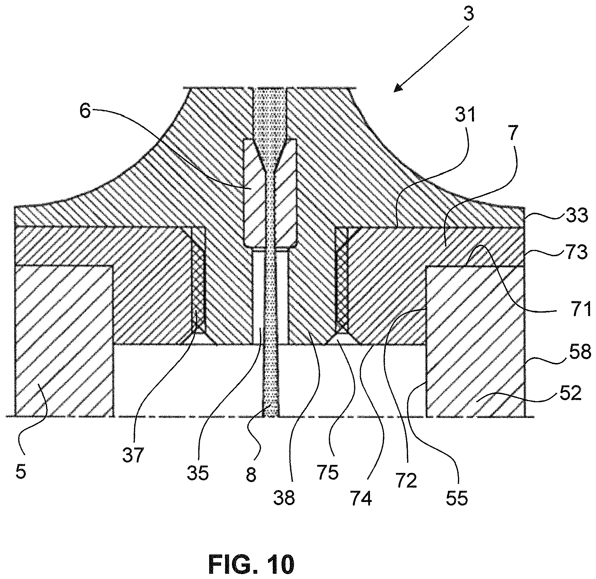

Die Figur 10 zeigt eine weitere Alternative zur lösbaren Verbindung vom vorderen Ende 3 mit dem porösen Körper 5. Der poröse Körper 5 ist mittels eines Adapters 7 lösbar mit dem vorderen Ende 3 verbindbar. Vorzugsweise sind der Adapter 7 und das vordere Ende 3 transparent ausgebildet. Das vordere Ende 3 weist einen Verbindungszapfen 38 auf, welcher zentral angeordnet ist und die Austrittsöffnung 35 umgibt. Der Adapter weist eine zentrale zweite Durchgangsöffnung 75 auf, welche koaxial mit dem porösen Körper 5 ausgerichtet ist. Der Verbindungszapfen 38 und die zweite Durchgangsöffnung 75 bilden zusammen eine lösbare Verbindung 37, hier dargestellt in der Form einer Schraubverbindung. Eine zweite vordere Stirnfläche 74 des Adapters 7 weist eine Aussparung mit einer zweiten axialen und einer zweiten radialen Begrenzungsfläche 71, 72 zur Aufnahme des porösen Körpers 5 auf. Der poröse Körper 5 liegt mit der Innenfläche 55 an der zweiten radialen Begrenzung 72 des Adapters 7 an und liegt mit der hinteren Stirnfläche 56 an der zweiten axialen Begrenzung 71 des Adapters 7 an. Die äussere Oberfläche 58 des porösen Körpers 5 ist fluchtend mit einer zweiten radialen Aussenfläche 73 des Adapters 7 ausgebildet.

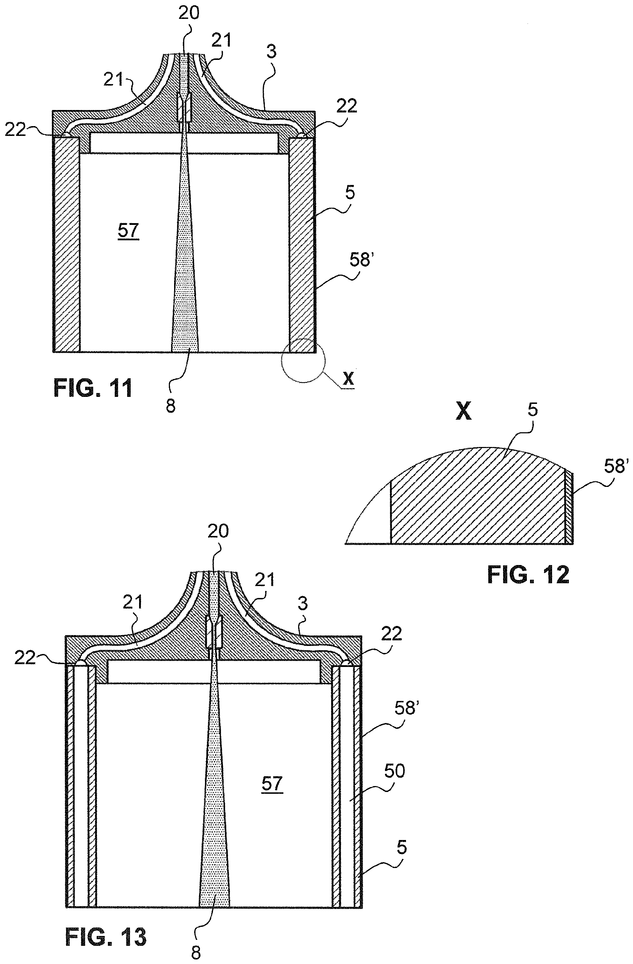

In Figur 11 ist eine Ausführungsform mit einer dichten Aussenhaut 58' und einer Absaugung vorgesehen. Die dichte Aussenhaut 58' ist in Figur 12 gut erkennbar. Sie ist vorzugsweise eine auf den porösen Körper aufgebrachte Schicht, welche den äusseren Umfang des porösen Körpers 5 vollständig umgibt und in luft- und flüssigkeitsdicht gegen aussen dichtet. Vorzugsweise bedeckt sie den Mantel, lässt jedoch die untere Stirnfläche 59 des porösen Körpers 5 frei, so dass diese offenporig ausgebildet ist. In alternativen Ausführungsformen bedeckt sich auch diese untere Stirnfläche.

Der Grundkörper 2 weist mindestens einen Saugkanal auf, welcher bzw. welche sich im vorderen Ende 3 des Handstücks 1 in Saugkanäle 21 aufteilen und zur oberen Stirnfläche des porösen Körpers führen. Es können auch bereits im Grundkörper 2 mehrere Saugkanäle 21 vorhanden sein, welche sich z.B. nicht weiter aufteilen. An dieser Stirnfläche ist vorzugsweise ein ringförmiger Verteilerkanal 22 vorhanden, welcher zum porösen Körper 5 hin offen oder abschnittsweise offen ausgebildet ist und welcher den über den Saugkanal angelegten Unterdruck gleichmässig über den stirnseitigen Umfang des porösen Körpers 5 verteilt. In dieser Ausführungsform erfolgt die Absaugung über die Poren des porösen Körpers 5.

In der Ausführungsform gemäss Figur 13 weist der poröse Körper 5 mehrere, vorzugsweise gleichmässig, über seinen Umfang verteilte Saugkanäle 50 auf. Diese Saugkanäle 50 verlaufen parallel zur Manteloberfläche und/oder parallel zur Strahlrichtung des Fluidstrahls 8. Die Saugkanäle 50 verlaufen vorzugsweise geradlinig und weisen einen grösseren Durchmesser als die durchschnittliche Porengrösse des porösen Körpers 5 auf. Vorzugsweise ist ihr Durchmesser ein Vielfaches der durchschnittlichen Porengrösse. Diese vertikalen Saugkanäle 50 erstrecken sich vorzugsweise bis zur unteren Stirnfläche 59 des porösen Körpers und sind somit nach unten offen ausgebildet. Sie können jedoch auch weiter oben enden oder mit der dichten Aussenhaut verschlossen sein.

Der Verteilerkanal 22 ist je nach Ausführungsform ausschliesslich zu diesen Saugkanälen 50 hin geöffnet oder er öffnet sich auch gegenüber anderen Stellen der Stirnfläche des porösen Körpers. Diese Ausführungsform mit den Saugkanälen 50 weist den Vorteil auf, dass die Absaugung auch bei stark gesättigten porösen Körper 5 noch funktioniert, da eine Verstopfung der Poren verhindert wird.

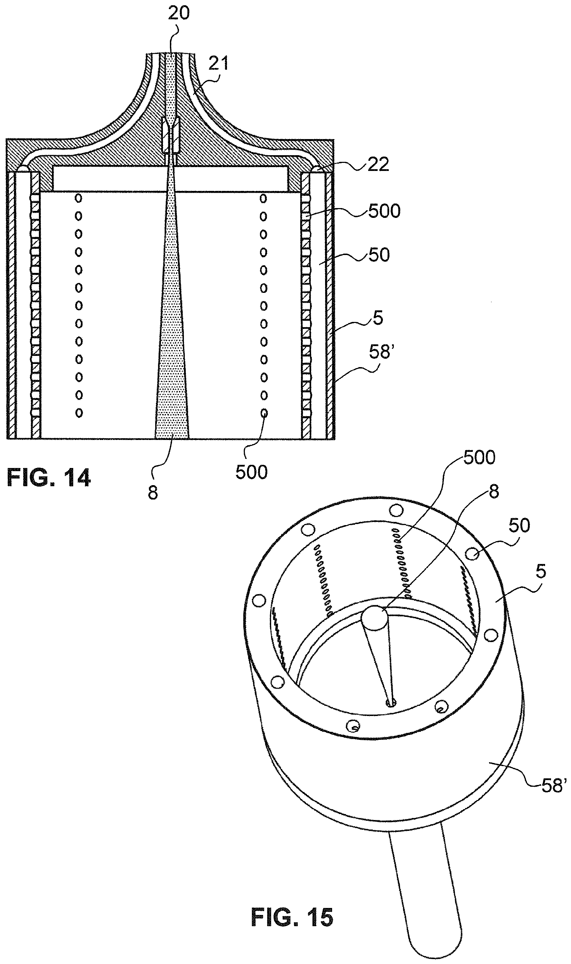

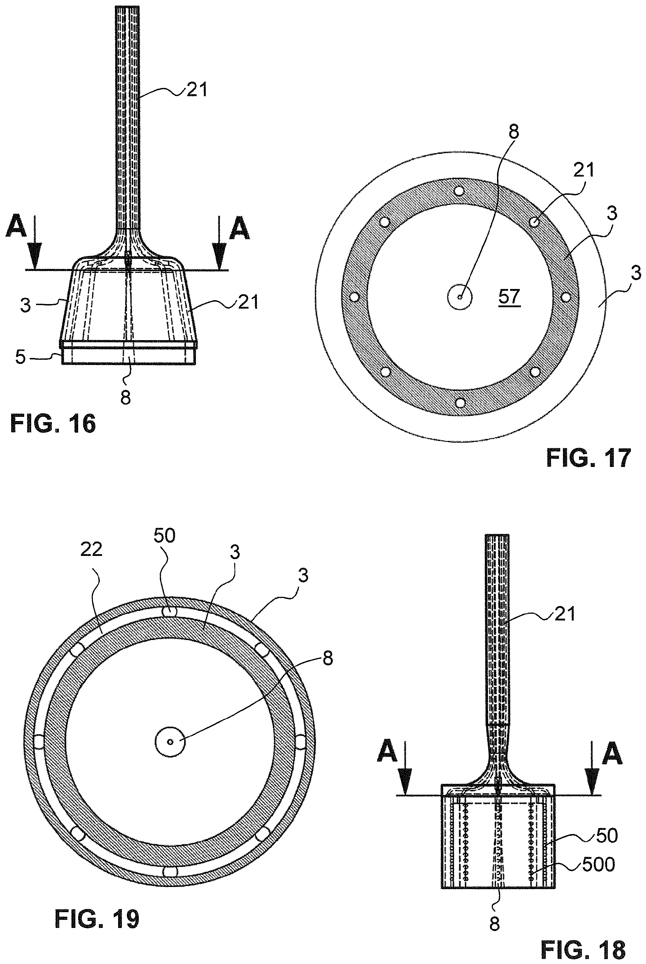

In der Ausführungsform gemäss den Figuren 14 und 15 sind zusätzlich zu den axial verlaufenden Saugkanälen 50 noch radial verlaufende Saugkanäle 500 vorhanden. Diese radial verlaufenden Saugkanäle 500 verbinden die axialen Saugkanäle 50 mit dem hohlen Innenraum 57 des porösen Körpers 5, d.h. den Raum, welchen der Fluidstrahl ungehindert passiert. Die radialen Saugkanäle 500 sind vorzugsweise verteilt über die gesamte Länge der axialen Saugkanäle 50 angeordnet. In den Figuren 18 und 19 ist der ringförmige Verteilerkanal 22 gut erkennbar, welcher zu den axialen Saugkanälen 50 führt.

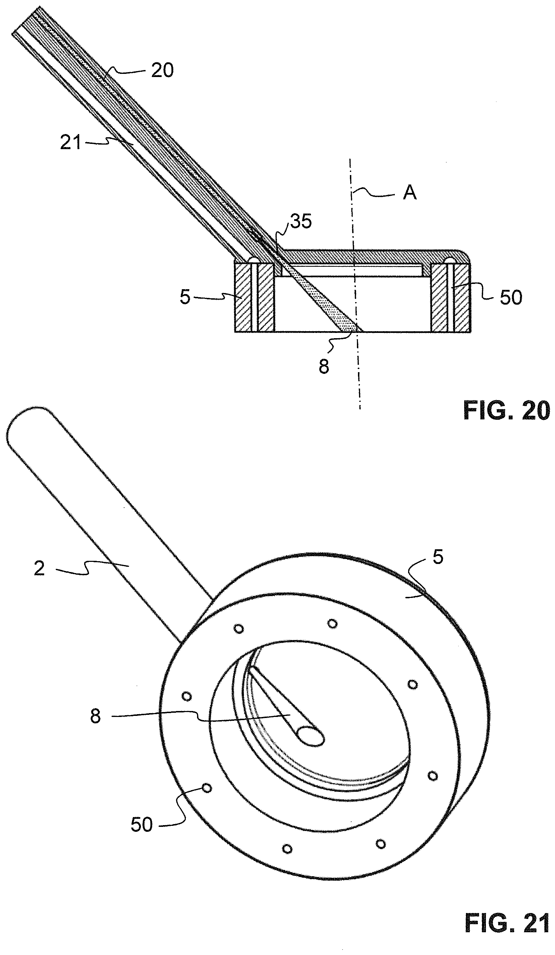

Die Figuren 16 und 17 zeigen eine weitere Ausführungsform. Die vom Grundkörper 2 her führenden Saugkanäle 21 erstrecken sich durch das vordere Ende 3 und münden direkt in den porösen Körper 5. Das vordere Ende 3 ist relativ gross ausgebildet und weist vorzugsweise eine sich zum freien Ende hin erweiternde konische Form auf. Der daran befestigte poröse Körper 5 ist hier zylindrisch geformt und in Strahlrichtung relativ kurz ausgebildet.
In der Ausführungsform gemäss den Figuren 20 und 21 sind die Austrittsöffnung 35 sowie der Fluidkanal 20 schräg zur Längsmittelachse A ausgerichtet, so dass der austretende Fluidstrahl in einem Winkel zur Längsmittelachse A verläuft. Vorzugsweise beträgt der Winkel annähernd 45°. Der hohle Innenraum des porösen Körpers 5 ist dabei vorzugsweise so gross bemessen, dass der Fluidstrahl nicht auf eine Innenwand des Körpers auftrifft. Es kann eine Absaugung vorhanden sein. In anderen Ausführungsformen ist keine Absaugung vorhanden. Zudem kann der poröse Körper wie dargestellt einen oder mehrere axiale und/oder radiale Saugkanäle aufweisen. In anderen Ausführungsformen weist er keine Saugkanäle auf. Im übrigen lassen sich die für die anderen Ausführungsformen beschriebenen Merkmale auch bei einer winkligen Anordnung des Fluidstrahls einsetzen. So lassen sich insbesondere die oben beschriebenen porösen Körper mit ihren Formen und Saugkanälen auch in dieser Ausführungsform einsetzen. In diesem Beispiel ist lediglich ein einziger Saugkanal 21 im Handstück 2 vorhanden, welcher in den ringförmigen Verteilerkanal 22 mündet. Es lassen sich jedoch auch hier mehrere Saugkanäle 21 im Handstück 2 anordnen.

Das erfindungsgemässe Handstück kombiniert die Vorteile einer Reinigung mit Fluidstrahl mit den Vorteilen einer mechanischen Reinigung und bietet gleichzeitig einen wirksamen Schutz vor Aerosolen.

**Bezugszeichenliste**

| | | | |
|---|---|---|---|
| 1 | Handstück | 53 | Vordere Auskragung |
| 2 | Grundkörper | 54 | Hintere Auskragung |
| 20 | Fluidkanal | 55 | Innenfläche |
| 21 | Saugkanäle | 56 | Hintere Stirnseite |
| 22 | Verteilerkanal | 57 | Erste Durchgangsöffnung |
| 3 | Vorderes Ende | 58 | Äussere Oberfläche |
| 31 | Erste axiale Begrenzung | 58' | Aussenhaut |
| 32 | Erste radiale Begrenzung | 59 | Kontaktfläche |
| 33 | Erste radiale Aussenfläche | 6 | Düse |
| 34 | Erste vordere Stirnfläche | 60 | Düsenkanal |
| 35 | Austrittsöffnung | 7 | Adapter |
| 36 | Vorderer Anschlag | 71 | Zweite axiale Begrenzung |
| 37 | Lösbare Verbindung | 72 | Zweite radiale Begrenzung |
| 38 | Verbindungszapfen | 73 | Zweite radiale Aussenfläche |
| 4 | Hinteres Ende | 74 | Zweite vordere Stirnfläche |
| 5 | Poröser Körper | 75 | Zweite Durchgangsöffnung |
| 50 | axialer Saugkanal | 8 | Fluidstrahl |
| 500 | radialer Saugkanal | 9 | Fluidleitung |
| 51 | Kontaktbereich | A | Längsmittelachse |
| 52 | Hinterer Bereich | | |

## Patentansprüche

1. Handstück (1) zum Reinigen von Wunden durch einen Fluidstrahl (8), umfassend einen Grundkörper (2) mit einem vorderen Ende (3) mit einer Austrittsöffnung (35) zum Austritt des Fluidstrahls (8), wobei am vorderen Ende (3) ein poröser Körper (5) vorhanden ist, welcher die Austrittsöffnung (35) umgibt und ihr in Richtung des Fluidstrahlauslasses vorsteht und welcher einen Raum bildet, der vom Fluidstrahl ungehindert passierbar ist, **dadurch gekennzeichnet, dass** im vorderen Ende (3) eine Düse (6) angeordnet ist, welche die Austrittsöffnung (35) bildet und dass das Handstück (1) steif ausgebildet ist.

2. Handstück (1) gemäss Anspruch 1, wobei der poröse Körper (5) aus einem schwamm-, vlies- oder gewirkeartigen Material besteht.

3. Handstück (1) gemäss einem der Ansprüche 1 oder 2, wobei der poröse Körper (5) mit einer Innenfläche (55) und/oder mit einer dem vorderen Ende (3) zugewandten hinteren Stirnseite (56) am vorderen Ende (3) anordnungsbar ist.

4. Handstück (1) gemäss einem der Ansprüche 1 bis 3, wobei der poröse Körper (5) fix oder lösbar am vorderen Ende (3) angeordnet ist.

5. Handstück (1) gemäss einem der Ansprüche 1 bis 4, wobei das vordere Ende (3) eine Aussparung mit einer ersten axialen und einer ersten radialen Begrenzungsfläche (31, 32) zur Aufnahme des porösen Körpers (5) aufweist.

6. Handstück (1) gemäss einem der Ansprüche 1 bis 5, wobei der poröse Körper (5) mittels eines Adapters (7) am vorderen Ende (3) anordnungsbar ist.

7. Handstück (1) gemäss einem der Ansprüche 1 bis 6, wobei der poröse Körper (5) eine im Wesentlichen hohlzylindrische, hohlkeglige, oder hohl-polyedrische Form aufweist.

8. Handstück (1) gemäss einem der Ansprüche 1 bis 7, wobei der poröse Körper (5) einen Kontaktbereich (51) zum Kontakt mit einer Wunde aufweist, welcher sich im Wesentlichen senkrecht zur Richtung des Fluidstrahlauslasses oder welcher sich im Wesentlichen unter einem Winkel ungleich 90° zur Richtung des Fluidstrahlauslasses erstreckt.

9. Handstück (1) gemäss einem der Ansprüche 1 bis 8, wobei der poröse Körper (5) im Kontaktbereich (51) eine nach aussen gerichtete, umlaufende vordere Auskragung (53) aufweist.

10. Handstück (1) gemäss einem der Ansprüche 1 bis 9, wobei der poröse Körper (5) in einem hinteren Bereich (52) eine nach innen gerichtete, umlaufende hintere Auskragung (54) zum Eingreifen in eine entsprechende Ausnehmung (37) des vorderen Endes (3) aufweist.

11. Handstück (1) gemäss einem der Ansprüche 1 bis 10, wobei der poröse Körper (5) einen äusseren Mantel aufweist, welcher eine dichte Aussenhaut (58') bildet.

12. Handstück (1) nach einem der Ansprüche 1 bis 11, wobei im porösen Körper (5) Saugkanäle (50, 500) vorhanden sind, welche einen Durchmesser aufweisen, der um ein Vielfaches grösser sind als eine durchschnittliche Porengrösse seiner Poren.

13. Handstück (1) nach Anspruch 12, wobei sich die Saugkanäle (50) in axialer Richtung erstrecken und geradlinig verlaufen.

14. Handstück (1) nach einem der Ansprüche 12 oder 13, wobei radial verlaufende Saugkanäle (500) vorhanden sind, welche in den durch den porösen Körper (5) gebildeten Raum (57) münden.

15. Handstück (1) nach einem der Ansprüche 1 bis 14, wobei die Austrittsöffnung (35) den Fluidstrahl (8) in einer Richtung austreten lässt, welche in einem Winkel zu einer Längsmittelachse des porösen Körpers (5) verläuft.

## Claims

1. A handpiece (1) for cleansing wounds with a fluid jet (8), said handpiece (1) comprising a main body (2), which has a front end (3) with an emergence opening (35) for the emergence of the fluid jet (8), wherein a porous body (5) is present on the front end (3), which porous body (5) surrounds the emergence opening (35) and protrudes beyond the latter in the direction of the fluid jet outlet and forms a space through which the fluid jet can pass unimpeded, **characterized in that** a nozzle (6) is arranged in the front end (3) and forms the emergence opening (35), and **in that** the handpiece (1) is stiff.

2. The handpiece (1) as claimed in claim 1, wherein the porous body (5) is made of a sponge, fleece or knit like material.

3. The handpiece (1) as claimed in either of claims 1 and 2, wherein the porous body (5) can be arranged on the front end (3) via an inner surface (55) and/or via a rear face (56) directed toward the front end (3) .

4. The handpiece (1) as claimed in one of claims 1 through 3, wherein the porous body (5) is arranged in a fixed manner or releasably on the front end (3) .

5. The handpiece (1) as claimed in one of claims 1 through 4, wherein the front end (3) has a recess with a first axial limit surface (31) and a first radial limit surface (32) for receiving the porous body (5).

6. The handpiece (1) as claimed in one of claims 1 through 5, wherein the porous body (5) can be arranged on the front end (3) by means of an adapter (7).

7. The handpiece (1) as claimed in one of claims 1 through 6, wherein the porous body (5) has a substantially hollow cylindrical, hollow conical or hollow polyhedral shape.

8. The handpiece (1) as claimed in one of claims 1 through 7, wherein the porous body (5) has a contact area (51) for contact with a wound, which contact area (51) extends substantially perpendicularly with respect to the direction of the fluid jet outlet or extends substantially at an angle other than 90° with respect to the direction of the fluid jet outlet.

9. The handpiece (1) as claimed in one of claims 1 through 8, wherein the porous body (5) has, in the contact area (51), an outwardly directed, circumferential front collar (53).

10. The handpiece (1) as claimed in one of claims 1 through 9, wherein the porous body (5) has, in a rear area (52), an inwardly directed, circumferential rear collar (54) for engaging in a corresponding recess (37) of the front end (3).

11. The handpiece (1) as claimed in one of claims 1 through 10, wherein the porous body (5) has an outer jacket which forms a tight outer skin (58').

12. The handpiece (1) as claimed in one of claims 1 through 11, wherein suction channels (50, 500) are present in the porous body (5) and have a diameter many times larger than an average pore size of its pores.

13. The handpiece (1) as claimed in claim 12, wherein the suction channels (50) extend in the axial direction and are rectilinear.

14. The handpiece (1) as claimed in either of claims 12 and 13, wherein radially extending suction channels (500) are present, which open into the space (57) formed by the porous body (5).

15. The handpiece (1) as claimed in one of claims 1 through 14, wherein the emergence opening (35) allows the fluid jet (8) to emerge in a direction which is at an angle with respect to a longitudinal central axis of the porous body (5).

## Revendications

1. Pièce à main (1) destinée à nettoyer des plaies par le biais d'un jet de fluide (8), la pièce à main comprenant un corps de base (2) pourvu d'une extrémité avant (3) dotée d'un orifice de sortie (35) destiné à la sortie du jet de fluide (8), un corps poreux (5) étant prévu à l'extrémité avant (3), lequel entoure l'orifice de sortie (35) et saillant depuis celui-ci en direction de la sortie du jet de fluide et forme un espace que le jet de fluide peut traverser sans entrave, **caractérisée en ce qu'**une buse (6) est disposée dans l'extrémité avant (3) et forme l'orifice de sortie (35) et **en ce que** la pièce à main (1) est rigide.

2. Pièce à main (1) selon la revendication 1, le corps poreux (5) étant formé à partir d'un matériau spongieux, non-tissé ou tricoté.

3. Pièce à main (1) selon l'une des revendications 1 ou 2, le corps poreux (5) pourvu d'une surface intérieure (55) et/ou d'un côté frontal extrémité arrière (56) tourné vers l'extrémité avant (3) pouvant être disposé à l'extrémité avant (3).

4. Pièce à main (1) selon l'une des revendications 1 à 3, le corps poreux (5) étant disposé de manière fixe ou amovible à l'extrémité avant (3).

5. Pièce à main (1) selon l'une des revendications 1 à 4, l'extrémité avant (3) comportant un évidement pourvu de premières surfaces limites axiale et radiale (31, 32) destinées à recevoir le corps poreux (5).

6. Pièce à main (1) selon l'une des revendications 1 à 5, le corps poreux (5) pouvant être disposé à l'extrémité avant (3) au moyen d'un adaptateur (7).

7. Pièce à main (1) selon l'une des revendications 1 à 6, le corps poreux (5) ayant sensiblement une forme de cylindre creux, de cône creux, ou de polyèdre creux.

8. Pièce à main (1) selon l'une des revendications 1 à 7, le corps poreux (5) comportant une région de contact (51) qui est destinée à venir en contact avec une plaie et qui s'étend sensiblement perpendiculairement à la direction de la sortie du jet de fluide ou sensiblement suivant un angle différent de 90° par rapport à la direction de la sortie du jet de fluide.

9. Pièce à main (1) selon l'une des revendications 1 à 8, le corps poreux (5) comportant dans la région de contact (51) une saillie avant circonférentielle (53) dirigée vers l'extérieur.

10. Pièce à main (1) selon l'une des revendications 1 à 9, le corps poreux (5) comportant dans une région arrière (52) une saillie arrière circonférentielle (54) dirigée vers l'intérieur et destinée à s'engager dans un évidement correspondant (37) de l'extrémité avant (3).

11. Pièce à main (1) selon l'une des revendications 1 à 10, le corps poreux (5) comportant une enveloppe extérieure qui forme une peau extérieure dense (58').

12. Pièce à main (1) selon l'une des revendications 1 à 11, des canaux d'aspiration (50, 500) étant ménagés dans le corps poreux (5), lesquels ont un diamètre qui est un multiple de la taille moyenne des pores de celui-ci.

13. Pièce à main (1) selon la revendication 12, les canaux d'aspiration (50) s'étendant dans la direction axiale et en ligne droite.

14. Pièce à main (1) selon l'une des revendications 12 ou 13, des canaux d'aspiration radiaux (500) étant prévus qui débouchent dans l'espace (57) formé par le corps poreux (5).

15. Pièce à main (1) selon l'une des revendications 1 à 14, l'orifice de sortie (35) permettant au jet de fluide (8) de sortir dans une direction qui fait un angle avec un axe central longitudinal du corps poreux (5).
